# EUROPEAN PATENT APPLICATION

(11) **EP 3 427 595 A1**
(43) Date of publication of application: **16.01.2019**
(21) Application number: 17763332.8
(22) Date of filing: 08.03.2017
(51) Int. Cl.: A23J 3/16, A23J 3/14, A23L 13/00, A23L 35/00, A23L 11/00, A23L 33/185, C12N 9/04, C12N 9/10, C12N 9/16

(54) **METHOD FOR PRODUCING VEGETABLE PROTEIN-CONTAINING FOOD**

(30) Priority: 10.03.2016 JP 2016047334
(71) Applicant: Ajinomoto Co., Inc., Tokyo 104-8315 (JP)
(72) Inventor: TOHO, Yuki, Kawasaki-shi Kanagawa 210-8681 (JP); OSANAI, Nao, Kawasaki-shi Kanagawa 210-8681 (JP); SATO, Hiroaki, Kawasaki-shi Kanagawa 210-8681 (JP)
(74) Representative: Strehl Schübel-Hopf & Partner
(86) International application number: PCT/JP2017/009297
(87) International publication number: WO 2017/154992

(57) **Abstract**

Provided is a vegetable protein-containing food having an excellent texture, preferably also having good flavor/taste. The vegetable protein-containing food can be produced by adding transglutaminase and glucose oxidase or adding transglutaminase, glucose oxidase and phospholipase to a food raw material containing a vegetable protein.

## Description

### Technical Field

The present invention relates to a method for producing a plant protein-containing food such as meat substitute foods.

### Background Art

In recent years, there are increasing demands for plant protein-containing foods obtained by processing plant proteins such as soybean protein. Such plant protein-containing foods are usually processed to have fibrous texture in order to impart a meat-like mouthfeel to the foods as meat substitute foods. As methods for texturizing proteins, methods utilizing a biaxial extruder are mainly used, and for example, dry soybean protein processed by such methods is marketed as granular soybean protein. However, mouthfeel thereof is slightly different from that of meat, and tends to lack elasticity and juiciness much or less.

Usual Hamburg steaks are foods obtained by binding minced beef or pork using wheat flour, breadcrumbs, or egg. However, in cases of Hamburg steak type foods using plant proteins, it has been difficult to find a plant-derived ingredient suitable as a binding material (binder) for putting granular soybean protein as a substitute for minced meat together in the shape of Hamburg steak. As techniques for improving mouthfeel of plant protein-containing foods, there are known a method of adding a sugar alcohol to a texturized granular plant protein material (Patent document 1), and a method for binding a meat substitute comprising processing a sample consisting of a mixture of granular soybean protein, isolated soybean protein, and wheat flour into a minced state with an extruder to obtain a texturized protein, molding the texturized protein with mixing, and irradiating microwaves on the molded texturized protein (Patent document 2).

However, the aforementioned techniques still have rooms for improvement in regard of mouthfeel of products and ease of the operations.

As techniques for modifying foods, methods using an enzyme have been developed. For example, transglutaminase is widely used as an enzyme for binding food materials. Specifically, for example, there is known a technique of imparting elasticity and stickiness to noodles by using a transglutaminase in combination with a carbonate and/or a reducing agent such as glutathione (Patent document 3). There is also disclosed a method for producing a soybean protein gel comprising allowing a protein crosslinking enzyme to act on a material containing a soybean protein material, and heating the material, and transglutaminase is used as the protein crosslinking enzyme in this method (Patent document 4).

There is further known a method for producing a food such as rice foods and noodles by using transglutaminase in combination with glucose oxidase and/or α-glucosidase (Patent document 5).

It is known that phospholipase can be used for preventing generation of air bubbles in bread (Patent document 6), and for manufacture of bread having puffy volume (Patent document 7).

However, it is not known that combinatory use of transglutaminase and glucose oxidase, or combinatory use of these enzymes and phospholipase can improve mouthfeel as well as flavors and tastes of foods containing plant proteins such as soybean protein.

### Prior art references

### Patent documents

Patent document 1: Japanese Patent No. 5198687
Patent document 2: Japanese Patent Laid-open (KOKAI) No. 2008-61592
Patent document 3: Japanese Patent Laid-open (KOKAI) No. 10-262588
Patent document 4: Japanese Patent No. 5577702
Patent document 5: Japanese Patent Laid-open (KOKAI) No. 2011-206048
Patent document 6: Japanese Patent Laid-open (KOKAI) No. 08-266213
Patent document 7: Japanese Patent Laid-open (KOKAI) No. 2015-104343

### Summary of the Invention

### Object to be Achieved by the Invention

An object of the present invention is to provide a method for producing a plant protein-containing food showing superior mouthfeel, and according to a preferred embodiment thereof, a method for producing a plant protein-containing food showing superior mouthfeel and having favorable flavor and taste.

### Means for Achieving the Object

The inventor of the present invention found that a plant protein-containing food showing superior mouthfeel can be produced by adding transglutaminase and glucose oxidase to a food raw material containing a plant protein, and flavor and taste of such a food can be made more favorable by further adding phospholipase to such a food raw material, and accomplished the present invention.

That is, the present invention can be embodied, for example, as follows.
(1) A method for producing a plant protein-containing food, the method comprising:
   adding a transglutaminase and a glucose oxidase to a food raw material containing a plant protein.
(2) The method mentioned above, which further comprises adding a phospholipase to the food raw material.
(3) The method mentioned above, wherein the phospholipase is phospholipase D.
(4) The method mentioned above, wherein the transglutaminase is added in an amount of 1.0 x 10⁻⁴ U or larger per 1 g of the food raw material, and the glucose oxidase is added in an amount of 1.0 x 10⁻⁴ U or larger per 1 g of the food raw material.
(5) The method mentioned above, wherein the phospholipase is added in an amount of 1.0 x 10⁻³ U or larger per 1 g of the food raw material.
(6) The method mentioned above,
   wherein the plant protein-containing food is a food prepared by mixing a binding material containing the food raw material containing a plant protein, with another food raw material in a crumble form, and cooking the mixture, and
   wherein the amounts of the transglutaminase and glucose oxidase to be added are read as amounts per 1 g of the binding material.
(7) The method mentioned above,
   wherein the plant protein-containing food is a food prepared by mixing a binding material containing the food raw material containing a plant protein, with another food raw material in a crumble form, and cooking the mixture, and
   wherein the amounts of the transglutaminase, glucose oxidase, and phospholipase to be added are read as amounts per 1 g of the binding material.
(8) The method mentioned above, wherein the plant protein is a soybean protein.
(9) The method mentioned above, wherein the plant protein-containing food is a meat substitute food.
(10) The method mentioned above, wherein the plant protein-containing food is a Hamburg steak type food.
(11) An enzyme preparation for producing a plant protein-containing food, the preparation containing a transglutaminase and a glucose oxidase.
(12) The enzyme preparation mentioned above, wherein the plant protein-containing food is a meat substitute food.
(13) The enzyme preparation mentioned above, which further contains a phospholipase.

### Best Mode for Carrying out the Invention

Hereafter, the present invention will be explained in detail.

The present invention provides a method for producing a plant protein-containing food, which method comprises adding a transglutaminase and a glucose oxidase to a food raw material containing a plant protein. The term "plant protein" refers to a protein derived from a plant, or a composition containing such a protein, and may be a product obtained by processing a plant to increase the protein content thereof. Specific examples of the plant protein include, for example, soybean protein. The term "food raw material" refers to a raw material used for producing a food.

The plant protein-containing food produced by the method of the present invention may be a final product, or may be a food material for producing a food. In this description, the term "food material" refers to a material produced from a food raw material, and mainly refers to an intermediate material used for producing a final product. Both a food material and a food raw material for producing the food material are regarded as a food raw material of the final product to be produced from the food material. The terms of food raw material, food material, and final product represent relative concepts. For example, a meat substitute (meat analogue, also referred to as substitutional meat or meat-like food), which is composed mainly of a plant protein, may be a final product produced from a plant protein as a raw material, then distributed and sold as it is, or may also be a food material to be used for producing a Hamburg type food as a final product (namely, food raw material of the Hamburg type food). Examples of the meat substitute include texturized soybean protein, i.e., so-called soybean meat (soy meat). Specific examples of the texturized soybean protein include, for example, a product obtained by texturizing soybean protein of which the protein content has been increased to 50% by weight or higher by processing soybeans. Examples of the final product include, but are not particularly limited to, meat substitute foods. In the present invention, the term "meat substitute food" refers to a food that should originally contain meat as at least a part thereof, but contains a meat substitute instead of meat. Examples of the meat substitute food include Hamburg steak type foods, meatball type foods, filling-wrapping dough sheet foods such as Chinese meat dumplings and steamed meat dumplings, meat sauce type foods, and so forth, and also include meat substitutes themselves. In these foods, a part or all of the meat may be replaced with a meat substitute. That is, these foods may contain, for example, both meat and a meat substitute.

The food raw material containing a plant protein may be a plant protein itself, or may be a food raw material preferably containing 3% by weight or more, more preferably 6% by weight or more, further preferably 10% by weight or more, of a plant protein. Examples of such a food raw material include granular plant protein, texturized plant protein, and so forth. Specific examples of the texturized plant protein include, for example, texturized soybean protein. The food raw material containing a plant protein may consist of a plant protein, or may further contain another ingredient such as a seasoning ingredient and water.

The food raw material used for the present invention or the food to be produced according to the present invention need not contain any raw material other than the plant protein, such as meat, albumen, wheat flour, and breadcrumbs, but may contain such a raw material so long as the effect of the present invention is not degraded. A preferred example of the food produced according to the present invention is a meat substitute food in which 50% by weight or more, preferably 80% by weight or more, more preferably all, of meat is replaced with a meat substitute.

A transglutaminase and a glucose oxidase are added to such a food raw material containing a plant protein as mentioned above. According to an embodiment of the present invention, a phospholipase is further added in addition to these enzymes. These enzymes may be simultaneously added to the food raw material, or may be successively added in an arbitrary order. The enzymes may be added to a part of the food raw material, and then the remaining portion of the food raw material may be added to the mixture. If the enzymes are added to the food raw material, the enzymes act on the food raw material. Therefore, "to add an enzyme to a food raw material" is synonymous with "to allow an enzyme to act on a food raw material".

The term "transglutaminase" refers to an enzyme having an activity for catalyzing an acyl group transfer reaction using a glutamine residue in a protein as a donor, and a lysine residue in a protein as a receptor. As transglutaminase, there are known those derived from various origins such as mammals, fishes, and microorganisms, and any of these may be used. Specific examples of the transglutaminase include the transglutaminase preparation derived from a microorganism and marketed with the brand name of "Activa (registered trademark) TG" from Ajinomoto Co., Inc.

The term "glucose oxidase" refers to an enzyme that catalyzes the reaction that generates gluconolactone (gluconolactone is non-enzymatically hydrolyzed into gluconate) and hydrogen peroxide by using glucose and oxygen as substrates. As the glucose oxidase, there are known those derived from various origins such as microorganisms including *Aspergillus* bacteria, and plants, and any of these may be used. Specific examples of the glucose oxidase include the glucose oxidase derived from a microorganism and marketed with the brand name of "Sumizyme PGO" from Shin Nihon Kagaku Kogyo Co., Ltd.

The term "phospholipase" refers to an enzyme that hydrolyzes a phospholipid into an aliphatic acid and another oleophilic substance. Phospholipase is roughly classified into those of types A, B, C, and D depending on the type of the reaction catalyzed thereby. Although any kind of phospholipase can be used in the present invention, phospholipase C that cleaves a phospholipid at a position immediately adjacent to a phosphoric acid ester group, and phospholipase D that cleaves a phosphoric acid ester bond to generate phosphatidic acid and an alcohol are preferred examples, and phospholipase D is a more preferred example. Examples of the phospholipase D include phospholipases D derived from plants such as phospholipase D derived from cabbage, phospholipases D derived from microorganisms such as phospholipase D produced by an actinomycete belonging to the genus *Streptomyces,* and so forth.

As for the addition amounts of the aforementioned enzymes, it is preferred that the addition amounts of the transglutaminase is 1.0 x 10⁻⁴ U or larger, preferably 1.0 x 10⁻³ U or larger, more preferably 1.0 x 10⁻² U or larger, per 1 g of the food raw material. It is preferred that the addition amounts of the glucose oxidase is 1.0 x 10⁻⁴ U or larger, preferably 1.0 x 10⁻³ U or larger, more preferably 1.0 x 10⁻² U or larger, per 1 g of the food raw material. It is preferred that the addition amounts of the phospholipase is 1.0 x 10⁻³ U or larger, preferably 1.0 x 10⁻² U or larger, more preferably 1.0 x 10⁻¹ U or larger, per 1 g of the food raw material.

Although the maximum addition amounts of the enzymes are not particularly limited, the addition amount of each of the transglutaminase, phospholipase, and glucose oxidase may usually be 100 U or smaller per 1 g of the food raw material.

The enzymatic activities can be measured as follows.

The following method can be exemplified as the method for measuring the enzymatic activity of transglutaminase. A transglutaminase is allowed to act on benzyloxycarbonyl-L-glutaminylglycine and hydroxylamine as substrates to generate hydroxamic acid. Hydroxamic acid is made into an iron complex in the presence of trichloroacetic acid, then absorbance is measured at 525 nm, the amount of hydroxamic acid is obtained by using a calibration curve, and the enzymatic activity is calculated. The amount of the enzyme that produces 1 µmol of hydroxamic acid in 1 minute at 37°C and pH 6.0 is defined as 1 U (1 unit).

The following method can be exemplified as the method for measuring the enzymatic activity of glucose oxidase. A glucose oxidase is allowed to act on glucose as a substrate in the presence of oxygen to generate hydrogen peroxide. Peroxidase is allowed to act on the generated hydrogen peroxide in the presence of aminoantipyrine and phenol to generate a quinoneimine dye. Absorbance is measured at 500 nm, the amount of the quinoneimine dye is obtained by using a calibration curve, and the enzymatic activity is calculated. The amount of the enzyme that oxidizes 1 µmol of glucose in 1 minute at 37°C and pH 7.0 is defined as 1 U (1 unit).

The following method can be exemplified as the method for measuring the enzymatic activity of phospholipase. A phospholipase is allowed to react on phosphatidylcholine as a substrate to release choline, and a choline oxidase is allowed to act on the released choline to generate hydrogen peroxide. A peroxidase is allowed to react on the generated hydrogen peroxide to allow quantitative oxidation condensation of N-ethyl-N-(2-hydroxy-3-sulfopropyl)-3,5-dimethoxyaniline sodium salt (DAOS) and 4-aminoantipyrine. Absorbance is measured at 600 nm, the amount of the oxidation condensate is obtained by using a calibration curve, and the enzymatic activity is calculated. The amount of the enzyme that releases 1 µmol of choline in 1 minute at 37°C and pH 5.5 is defined as 1 U (1 unit).

The aforementioned enzymes are added at least to a food raw material containing a plant protein. The aforementioned enzymes may be added to, for example, only a food raw material containing a plant protein, or a mixture of such a food raw material and another food raw material used for producing a food.
According to an embodiment of the present invention, a plant protein-containing food is produced by mixing a binding material containing a food raw material containing a plant protein, with another crumble-form food raw material, and cooking the mixture. The binding material is also called "binder". For example, a Hamburg steak type food using soybean protein (soybean Hamburg steak) is typically produced by using granular soybean protein, a binding material containing powdery soybean protein for binding the granular soybean protein, and another food raw material such as vegetables. In such a case, the enzymes are preferably added to the binding material. In this case, the expression "per 1 g of the food raw material" may mean "per 1 g of the final food product", but it preferably means "per 1 g of the binding material". The binding material may consist of a food raw material containing a plant protein, or may further contain other ingredients such as seasoning ingredients and water.

In order to allow the aforementioned enzymes to act on a food raw material, it is preferred that a fraction containing the food raw material (for example, the food raw material, a binding material containing the food raw material, or a mixture of the food raw material or such a binding material and another food raw material) contains 20 to 90% by weight, preferably 40 to 80% by weight, more preferably 60 to 80% by weight, of moisture.

The food raw material to which the enzymes have been added may be left as it is before the following production step, as required. Specifically, the food raw material to which the enzymes have been added may be, for example, kneaded, molded, and left as it is before the following production step, as required. It is preferred that the leaving period is 1 minute or longer, preferably 5 minutes to 24 hours, more preferably 30 minutes to 12 hours, and the leaving temperature is 0 to 80°C, preferably 10 to 60°C, more preferably 20 to 50°C.

A plant protein-containing food (for example, a final product) can be produced from a food raw material to which the enzymes have been added through a predetermined production step. Specifically, for example, the food raw material on which the enzymes have been acted can be mixed with another food raw material, processed, and further heat-cooked, as required, to thereby produce a plant protein-containing food (for example, a final product). The production of a food according to the present invention can be carried out in the same manner as a method usually used for the production of the corresponding food except that the enzymes are added to the food raw material containing a plant protein, and the mixture is left as it is as required. The food raw material used for the production of a plant protein-containing food may be the same as a food raw material usually used for the production of the corresponding food except that the food raw material containing a plant protein is used. Although the food raw material containing a plant protein is mainly used instead of meat, it may also be used together with meat. The food raw material containing a plant protein may also be added to a food that does not usually contain meat.

The plant protein-containing food produced by the method of the present invention may be frozen in a raw state or after heat cooking. Examples of the heat cooking include such methods as baking, stewing, and steaming.

Hereafter, the method for producing the plant protein-containing food will be explained by exemplifying the case of a soybean Hamburg steak. Crumbled soybean (minced meat-like soybean) is prepared as a substitute for minced meat. Crumbled soybean can be prepared by rehydrating granular soybean protein with hot water, dehydrating it, and adding water or seasoning liquid to the granular soybean protein to allow it to absorb moisture. It is preferred that the addition amount of water or seasoning liquid is preferably 1 to 5 times, more preferably 2 to 4 times, of the amount of the dehydrated soybean protein in terms of weight ratio. A binding material is separately prepared by mixing texturized or powdery soybean protein, water or seasoning liquid, and the enzymes, and the mixture is left as it is. The addition amount of water or the seasoning liquid is preferably 1 to 5 times, more preferably 2 to 4 times, of the amount of the texturized or powdery soybean protein in terms of weight ratio.

The crumbled soybean, the binding material, and vegetables such as stir-fried onion are mixed, and molded into a patty. The leaving period and temperature are as described above. By heat-cooking this patty, a heated soybean Hamburg steak can be produced. The heat cooking may be baking on a frying pan or in an oven, or may be stewing.

Mouthfeel of a plant protein-containing food can be improved by the method of the present invention. Examples of the mouthfeel include granular feel, binding elasticity, and so forth. According to a preferred embodiment of the present invention, flavor and taste of a plant protein-containing food can also be improved. Examples of the flavor and taste include weakness of vegetable smells such as soybean smell, meat-like feel, juiciness, and so forth.

Another aspect of the present invention is a plant protein-containing food containing the followings: a food raw material containing a plant protein; a transglutaminase; and a glucose oxidase. The plant protein-containing food preferably further contains a phospholipase. Still another aspect of the present invention is a plant protein-containing food produced by allowing a transglutaminase and a glucose oxidase to act on a food raw material containing a plant protein. The plant protein-containing food is more preferably one obtained by further allowing a phospholipase to act on the food raw material. Still another aspect of the present invention is a plant protein-containing food produced by mixing a binding material containing a food raw material containing a plant protein with another crumble-form food raw material, and cooking the mixture, wherein the binding material has been added with a transglutaminase and a glucose oxidase. In a preferred embodiment of the plant protein-containing food, the binding material further has been added with a phospholipase. Still another aspect of the present invention is a method for improving mouthfeel, and/or flavor and taste of a plant protein-containing food, which method comprises adding a transglutaminase and a glucose oxidase to a food raw material containing a plant protein. A phospholipase is preferably further added to the food raw material. The food raw material and the enzymes used in the aforementioned aspects are the same as those described for the production method of the present invention.

The enzyme preparation of the present invention is an enzyme preparation for producing a plant protein-containing food, which preparation contains a transglutaminase and a glucose oxidase. The enzyme preparation preferably further contains a phospholipase. The enzyme preparation may be a mixture containing these enzymes, or may be a kit including these enzymes as separate two or three kinds of enzymes. The enzyme preparation of the present invention can be preferably used for the production of the plant protein-containing food of the present invention.

The enzyme preparation may contain a raw material other than the enzymes (henceforth also referred to as "other raw material") so long as the effect of the present invention is not degraded. As the other raw material, for example, those used by being blended in seasonings, foods, and drinks can be used. Examples of the other raw material include excipients such as glucose, dextrin, starch, modified starch, and reduced malt sugar, proteins such as plant proteins, gluten, albumen, gelatin, and casein, seasonings such as sodium glutamate, animal extracts, aquatic product extracts, protein hydrolysates, and protein partial decomposition products, alkaline agents (pH adjustor) such as sodium carbonate, potassium carbonate, and calcined calcium, chelating agents such as gluconate and citrate, oxidation or reduction agents such as sodium ascorbate, glutathione, and cysteine, other food additives such as alginic acid, brine, oil and fats, dyes, acidulants, and perfumes, and so forth. As the other raw material, one kind of raw material may be used, or two or more kinds of raw materials may be used in combination. The enzyme preparation can be produced by, for example, appropriately mixing the active ingredients with these other raw materials.

### Examples

Hereafter, the present invention will be more specifically explained with reference to examples.

Soybean Hamburg steaks were prepared with the composition mentioned in Table 1. In Table 1, the blending amounts are indicated in terms of weight %. The granular soybean protein and powdery soybean protein were produced by processing soybeans to increase the protein content thereof to 50% by weight or higher, and granulating or powdering them, and granular soybean protein produced by Fuji Oil Co., Ltd., and powdered isolated soy protein produced by Fuji Oil Co., Ltd. were used for them, respectively.

**Table 1**

| Raw materials | | Blending amounts |
|---|---|---|
| | Granular soybean protein | 18.7% |
| | Powdery soybean protein | 8.8% |
| | Stir-fried onion | 6.7% |
| Seasoning group | Sugar | 1.0% |
| | Umami seasoning | 0.1% |
| | Spice | 0.1% |
| | Salt | 0.8% |
| | Protein hydrolysate | 0.9% |
| | Canola oil | 2.9% |
| | Water | 60.0% |

The granular soybean protein was rehydrated with hot water, then dehydrated, allowed to absorb 40% by weight of the seasoning group mixture (including water), and thereby made into crumbled soybean.

Test samples were added to the total amount of the remaining seasoning group mixture, they were mixed, and the obtained mixture and the powdery soybean protein were mixed to obtain a binder. As the test samples, an enzyme solution or water was added in an amount of 0.1 % by weight based on the total amount of the raw materials mentioned in Table 1. The used enzymes were as follows. The enzymatic activities were measured by the methods described above, respectively. The amounts of the enzymes based on the product weight are shown in Table 2.

Transglutaminase (TG): "Activa (registered trademark) TG", Ajinomoto Co., Inc. Glucose oxidase (GO): "Sumizyme PGO", Shin Nihon Kagaku Kogyo Co., Ltd. Phospholipase D (PLD): "PLD", Nagase & Co., Ltd.

**Table 2**

| U/g based on product | TG | GO | PLD |
|---|---|---|---|
| Control | - | - | - |
| Comparative sample 1 | - | 1.4 x 10⁻³ U/g | - |
| Comparative sample 2 | - | 7.0 x 10⁻³ U/g | - |
| Comparative sample 3 | - | 3.5 x 10⁻² U/g | - |
| Comparative sample 4 | 1.3 x 10⁻³ U/g | - | - |
| Comparative sample 5 | 6.4 x 10⁻³ U/g | - | - |
| Comparative sample 6 | 3.2 x 10⁻² U/g | - | - |
| Test sample 1 | 1.3 x 10⁻³ U/g | 1.4 x 10⁻³ U/g | - |
| Test sample 2 | 6.4 x 10⁻³ U/g | 1.4 x 10⁻³ U/g | - |
| Test sample 3 | 2.2 x 10⁻² U/g | 2.4 x 10⁻² U/g | - |
| Test sample 4 | 3.2 x 10⁻² U/g | 7.0 x 10⁻³ U/g | - |
| Test sample 5 | 6.4 x 10⁻² U/g | 7.0 x 10⁻³ U/g | - |
| Test sample 6 | 3.2 x 10⁻² U/g | 3.5 x 10⁻² U/g | - |
| Test sample 7 | 6.4 x 10⁻² U/g | 7.0 x 10⁻² U/g | - |
| Test sample 8 | 6.4 x 10⁻² U/g | 7.0 x 10⁻² U/g | 0.275 U/g |
| Test sample 9 | 6.4 x 10⁻² U/g | 7.0 x 10⁻² U/g | 1.375 U/g |

The crumbled soybean, binder, and stir-fried onion prepared as described above were mixed. The mixture in an amount of 65 g was molded into a patty having a diameter of 8.0 cm and a thickness of 1 cm. A food processor (Bamix M300, Speed Cutter MK-K3, Matsushita Electric Industrial Co., Ltd.) was used for the mixing. The patty was incubated at 37°C for 1 hour, and baked at 216°C in a conveyer oven (Impinger, Lincoln Food Service Products, Inc.) for 4 minutes and 15 seconds for each side. The patty was completely cooled, then vacuum-packed by using a vacuum sealer (degree of vacuum 5500 Pa, sealing strength 1.8 s), and stored in a freezer. The frozen sample was thawed with running water, and then warmed on a hot plate at 160°C for 1.5 minutes for each side, and mouthfeel, and flavor and taste thereof were organoleptically evaluated by 4 persons of fully trained special panelists.

As for mouthfeel, meat granule-like mouthfeel of the crumbled soybean was defined as "granular feel", and shape-retaining strength of the putty was defined as "binding elasticity". As for flavor and taste, weakness of the soybean smell of the putty was defined as "weakness of soybean smell", intensity of meat-like flavor and taste was defined as "meat-like feel", and fat-like taste and stickiness were defined as "juiciness". A combination of higher scores of granular feel and binding elasticity means better meat-like mouthfeel. A combination of higher scores of granular feel and binding elasticity, and weaker soybean smell means better meat-like taste. Those items were evaluated according to the evaluation criteria mentioned in Table 3. Mouthfeel was evaluated for the control sample and test samples 1 to 7, and flavor and taste were further evaluated for the test samples 7 to 9. The results are shown in Tables 4 and 5.

**Table 3**

| | Score 0 | Score 1 | Score 2 | Score 3 | Score 4 |
|---|---|---|---|---|---|
| Granular feel | No difference from control | Slight granular feel | Granular feel | Strong granular feel | Extremely strong granular feel |
| Binding elasticity | No difference from control | Slightly elastic | Elastic | Strongly elastic | Extremely strongly elastic |
| Weakness of soybean smell | No difference from control | Slightly weak soybean smell | Weak soybean smell | Extremely weak soybean smell | No soybean smell |
| Meat-like feel | No difference from control | Slight meat-like feel | Meat-like feel | Strong meat-like feel | Extremely strong meat-like feel |
| Juiciness | No difference from control | Slightly juicy | Juicy | Strongly juicy | Extremely strongly juicy |

**Table 4**

| No. | Granular feel | Binding elasticity | Evaluation results |
|---|---|---|---|
| Control | 0 | 0 | Binding was sticky, and crumbled soybean was not elastic. |
| Comparative sample 1 | 0.25 | 0 | Crumbled soybean was slightly elastic. |
| Comparative sample 2 | 0.5 | 0 | Stickiness of binder was reduced, crumbled soybean was elastic, and shape-retaining property was improved. |
| Comparative sample 3 | 1 | 0 | Results were similar to those of Comparative sample 2. |
| Comparative sample 4 | 0 | 1 | No change in crumbled soybean was observed, but better shape-retaining property than Comparative samples 1 to 3 was observed. |
| Comparative sample 5 | 0 | 1.5 | Results were similar to those of Comparative sample 4, but stronger effects than Comparative sample 4 were observed. |
| Comparative sample 6 | 0 | 1.5 | Results were similar to those of Comparative sample 5. |
| Test sample 1 | 1 | 1 | More favorable balance of total mouthfeel than Test sample 3, and slight stickiness were observed. |
| Test sample 2 | 1 | 1 | Results were similar to those of Test sample 1. |
| Test sample 3 | 1 | 1.5 | Both binder and crumbled soybean were elastic, and slightly sticky. |
| Test sample 4 | 3 | 2 | Stronger granular feel than Test sample 1 was observed. |
| Test sample 5 | 3 | 2 | No significant difference compared with Test sample 4 was observed. |
| Test sample 6 | 3 | 3 | Granular feel of crumbled soybean was stronger than that of binder. |
| Test sample 7 | 4 | 4 | The product was elastic as a whole, and showed good balance. |

**Table 5**

| No. | *Gf | *Be | *Ws | *Mf | *Jc | Evaluation results |
|---|---|---|---|---|---|---|
| Control | 0 | 0 | 0 | 0 | 0 | Binding was sticky, and crumbled soybean was not elastic. |
| Test sample 7 | 4 | 4 | 0 | 0 | 0 | The sample was elastic as a whole, and showed good balance. |
| Test sample 8 | 4 | 4 | 3 | 2 | 3 | Juiciness was improved in PDL addition amount-dependent manner, and natural fatty feel was observed. |
| Test sample 9 | 4 | 4 | 4 | 3 | 4 | |

| | | | | | | |
|---|---|---|---|---|---|---|
| *Gf, Granular feel *Be, Binding elasticity *Ws, Weakness of soybean smell *Mf, Meat-like feel *Jc, Juiciness | | | | | | |

It was demonstrated that if transglutaminase and glucose oxidase are added to soybean Hamburg steaks, mouthfeel such as granular feel and binding elasticity is improved, and if phospholipase is further added, flavor and taste such as weakness of soybean smell, meat-like feel, and juiciness are also improved.

### Industrial Applicability

According to the present invention, a plant protein-containing food showing superior mouthfeel can be produced. According to a preferred embodiment, a plant protein-containing food showing favorable flavor and taste in addition to superior mouthfeel can be produced. The method of the present invention requires neither a special apparatus nor complicated steps, and thus is convenient. Even when a binding material is required for producing a plant protein-containing food, the food can be produced without using albumen, breadcrumbs, starch, or the like. The present invention is useful in the food production industry or food service industry.

## Claims

1. A method for producing a plant protein-containing food, the method comprising:
adding a transglutaminase and a glucose oxidase to a food raw material containing a plant protein.

2. The method according to claim 1, which further comprises adding a phospholipase to the food raw material.

3. The method according to claim 2, wherein the phospholipase is phospholipase D.

4. The method according to any one of claims 1 to 3, wherein the transglutaminase is added in an amount of 1.0 x 10⁻⁴ U or larger per 1 g of the food raw material, and the glucose oxidase is added in an amount of 1.0 x 10⁻⁴ U or larger per 1 g of the food raw material.

5. The method according to any one of claims 2 to 4, wherein the phospholipase is added in an amount of 1.0 x 10⁻³ U or larger per 1 g of the food raw material.

6. The method according to claim 4,
wherein the plant protein-containing food is a food prepared by mixing a binding material containing the food raw material containing a plant protein, with another food raw material in a crumble form, and cooking the mixture, and
wherein the amounts of the transglutaminase and glucose oxidase to be added are read as amounts per 1 g of the binding material.

7. The method according to claim 5,
wherein the plant protein-containing food is a food prepared by mixing a binding material containing the food raw material containing a plant protein, with another food raw material in a crumble form, and cooking the mixture, and
wherein the amounts of the transglutaminase, glucose oxidase, and phospholipase to be added are read as amounts per 1 g of the binding material.

8. The method according to any one of claims 1 to 7, wherein the plant protein is a soybean protein.

9. The method according to any one of claims 1 to 8, wherein the plant protein-containing food is a meat substitute food.

10. The method according to any one of claims 1 to 9, wherein the plant protein-containing food is a Hamburg steak type food.

11. An enzyme preparation for producing a plant protein-containing food, the preparation containing a transglutaminase and a glucose oxidase.

12. The enzyme preparation according to claim 11, wherein the plant protein-containing food is a meat substitute food.

13. The enzyme preparation according to claim 11 or 12, which further contains a phospholipase.
